# EUROPEAN PATENT APPLICATION

(11) **EP 2 478 901 A2**
(43) Date of publication of application: **25.07.2012**
(21) Application number: 12165032.9
(22) Date of filing: 13.02.2008
(51) Int. Cl.: A61K 31/35, A61K 31/355, A61K 31/70, A61P 25/00

(54) **Neurogenic compounds**

(30) Priority: 14.02.2007 US 901239 P
(62) Divisional of application: 08825828.0
(71) Applicant: Mars Incorporated, McLean, Virginia 22101-3883 (US); THE SALK INSTITUTE FOR BIOLOGICAL STUDIES, La Jolla California 92037-1099 (US)
(72) Inventor: Hammerstone, John F, Rockville, MD Maryland 20850 (US); Kelm, Mark A, Madera, CA California 93637 (US); Gage, Fred H, La Jolla, CA California 92037 (US); Van Praag, Henriette, San Diego, CA California 92124 (US)
(74) Representative: Keen, Celia Mary

(57) **Abstract**

The invention relates to method(s) of use of the compound(s) described herein, e.g. method for stimulating neurogenesis, including *in vitro* neurogenesis, by contacting neuronal progenitor cells with an effective amount of the compound(s) described herein; method for treatment of a subject in need of treatment with a neurogenic compound; and/or for treatment of a disease or condition associated with damage to the hippocampus. The subject may be a human or a veterinary animal.

## Description

This invention was developed in part through funding provided by DARPA under grant no. DAAD-19-02-1-0267. The Federal Government may have rights in the invention.

### FIELD OF THE INVENTION

The invention relates to a composition comprising a neurogenic compound and a method of use of the neurogenic compound, e.g. for stimulating neurogenesis; for treatment of a subject in need of treatment with a neurogenic compound; and/or for treatment of a disease or condition associated with damage to the hippocampus.

### BACKGROUND OF THE INVENTION

The hippocampus is a part of the brain located inside the temporal lobe (humans have two hippocampi, one in each side of the brain). It forms a part of the limbic system and plays a role in memory and navigation.

The hippocampus plays an essential role in the formation of new memories about personally experienced events (episodic or autobiographical memory) and in spatial memory. Episodic memory (also called autobiographical memory) is defined as the explicit memory of events, including time, place, and associated emotions. It allows one to remember events that were personally experienced at a specific time and place. This episodic memory is the kind most often affected by various forms of amnesia (anterograde and retrograde). Spatial memory is defined as memory that relates to storing and processing spatial information. It plays a role in navigation and is confined to the hippocampus. The hippocampus, as part of a larger medial temporal lobe memory system, may also be responsible for general declarative memory (memory which can be explicitly verbalized-including, for example, memory for facts in addition to episodic memory). Some evidence exists that, although these forms of memory often last a lifetime, the hippocampus ceases to play a crucial role in the retention of the memory after a period of consolidation.

Damage to the hippocampus usually results in profound difficulties in forming new memories (anterograde amnesia), and normally also affects access to memories prior to the damage (retrograde amnesia). Although the retrograde effect normally extends some years prior to the brain damage, in some cases older memories remain suggesting that information that has been encoded in long-term memory for a lengthy period of time no longer requires the intervention of the hippocampus. Damage to the hippocampus can occur as a result of a variety of factors, for example, trauma, oxygen starvation (anoxia) and encephalitis. Hippocampal damage, for example hippocampal atrophy (where atrophy refers to the decreasing volume of the hippocampus), is also associated with certain forms of dementia. However, damage to the hippocampus does not affect certain aspects of memory such as the ability to learn new skills (playing a musical instrument, for example), suggesting that such abilities depend on a different type of memory (procedural memory) and different brain regions. There is evidence, for example, that a patient who has had his medial temporal lobes removed bilaterally as a treatment for epilepsy can still form new semantic memories.

Evidence also implicates the hippocampus in storing and processing spatial information. Without a fully functional hippocampus humans may not successfully remember the places they have been to and how to get where they are going. Researchers believe that the hippocampus plays a particularly important role in finding shortcuts and new routes between familiar places. Some people exhibit more skill at this sort of navigation than do others, and brain imaging shows that these individuals have more active hippocampi when navigating.

There is also evidence that the hippocampus is involved in depression. The hippocampus of a person who has suffered long-term clinical depression can be as much as twenty percent smaller than the hippocampus of someone who has never been depressed. Both long-term antidepressant treatment (at least a few weeks) and exercise can increase hippocampal neurogenesis and make up for previous volume loss, implying that increased neurogenesis improves symptoms of depression. With respect to antidepressant treatment, this effect does not occur if the drug is given for only a few days.

Factors which may stimulate hippocampal neurogenesis include physical activity, enriched environment, increased serotonin levels, electroconvulsive shock and/or caloric restriction. However, age, decreased serotonin levels, stress, opiates, amphetamines and/or glucorticoids may have the opposite effect of decreasing hippocampal neurogenesis.

Given the large number of subjects suffering from diseases or conditions that may benefit from neurogenic compounds, there is considerable interest in finding therapeutic ways to prevent and treat these diseases or conditions. The use of certain compounds for these applications is described herein.

### SUMMARY OF THE INVENTION

The invention relates to a composition comprising a neurogenic compound and a method of use of the neurogenic compound, e.g. for stimulating neurogenesis; for treatment of a subject in need of treatment with a neurogenic compound; and/or for treatment of a disease or condition associated with damage to the hippocampus.

In one aspect, the invention relates to a composition, such as a pharmaceutical, a food, a food additive, or a dietary supplement comprising the compound of the invention. The composition may optionally contain an additional therapeutic or beneficial-to-health agent, or may be administered in combination with another therapeutic or beneficial-to-health agent. Packaged products containing the above-mentioned composition and a label and/or instructions for use for stimulating neurogenesis; for treatment of a subject in need of treatment with a neurogenic compound; and/or for treatment of a disease or condition associated with damage to the hippocampus.

In another aspect, the invention relates to methods for stimulating neurogenesis; for treatment of a subject in need of treatment with a neurogenic compound; and/or for treatment of a disease or condition associated with damage to the hippocampus.

### BRIEF DESCRIPTION OF THE DRAWINGS

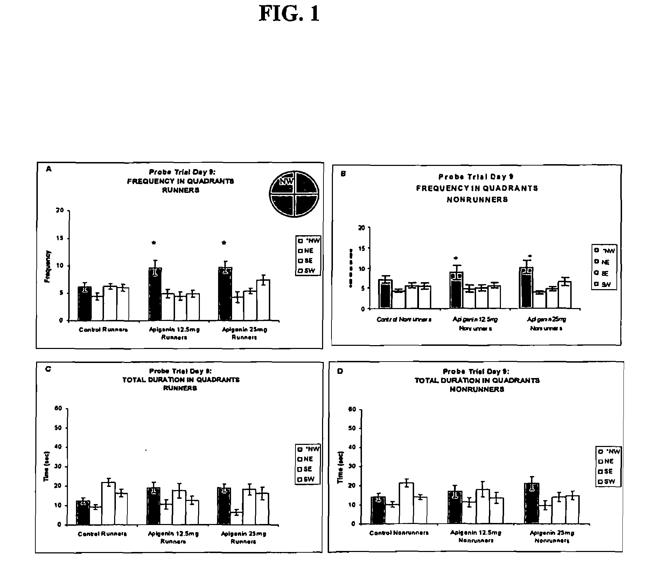

Figure 1A-D represents the results of the Morris Water Maze probe trial. Figures 1A and 1B show the frequency in quadrants for both runners (1A) and non-runners (1B). Figures 1C and 1D show the total duration in quadrants for both runners (1C) and non-runners (1D). The figures demonstrate that apigenin treated mice entered the target quadrant more frequently than all other quadrants.

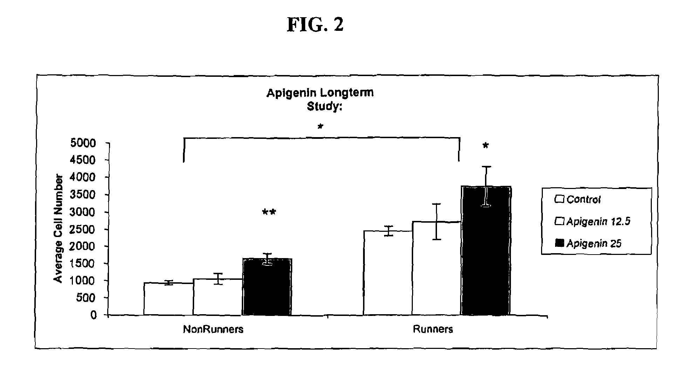

Figure 2 represents the total number of newly dividing cells (BrdU +) in the dentate gyrus. Apigenin increased BrdU labeling in both running and non-running groups. *p<.05 significantly different from control runners; **p<.01 significantly different from all other non-runners. p<.05 significantly different from running groups.

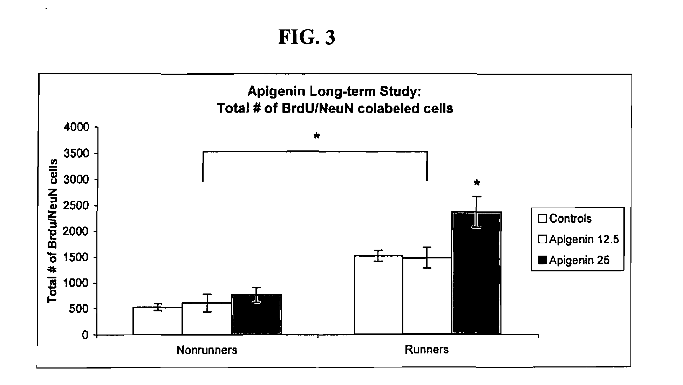

Figure 3 represents the total number of BrdU + cells co-labeled with NeuN in the dentate gyrus. Apigenin (25mg/kg) in combination with running resulted in the greatest increase in neurogenesis as compared to all other groups. *p<.05 significantly different from all other groups.

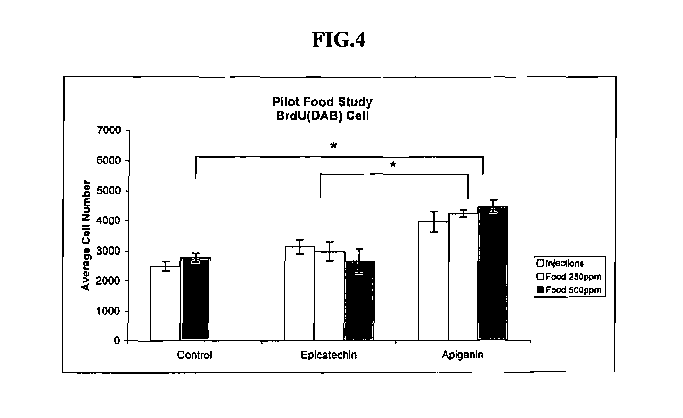

Figure 4 represents the total number of newly dividing cells (BrdU +) in the dentate gyrus. Both apigenin injection and food increased BrdU labeling as compared to all other groups. *p<.05 significantly different from apigenin.

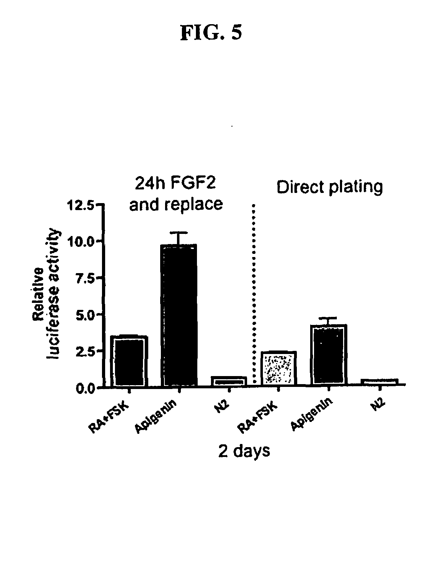

Figure 5 represents the quantification of relative NeuroD luciferase activity after a 2-day treatment under various conditions. Cells were first allowed to proliferate with RA + FSK, apigenin, or N2 alone (left). Cells were directly plated into N2 media containing RA + FSK, apigenin, or N2 only (right).

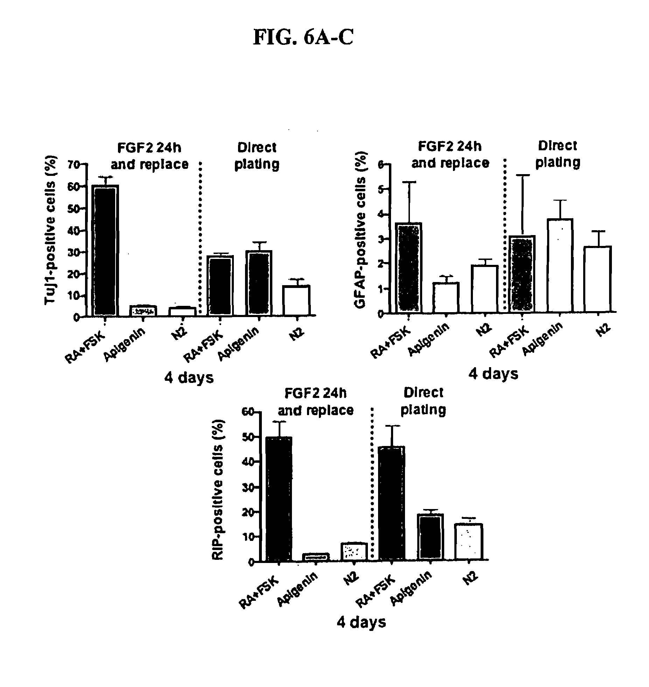

Figure 6 A-C represents the results of immunohistological analyses of apigenin-treated cells. Adult neural progenitor cells were disassociated and plated in N2 media containing FGF2 (left side of each graph) or N2 only (right side of each graph). Cells were grown for an additional four days after treatment with RA + FSK, apigenin, or N2 only, and then fixed and stained with lineage-specific markers (Tuj 1 for neurons, GFAP for astrocytes, and RIP for oliogodendrocytes). All counts are the number of marker-positive cells out of the whole (DAPI) positive cells. Error bars represent standard deviations.

### DETAILED DESCRIPTION

All patents, patent applications and references cited in this application are hereby incorporated herein by reference. In case of any inconsistency, the present disclosure governs.

The invention relates to a composition comprising a neurogenic compound and a method of use of the neurogenic compound, e.g. for stimulating neurogenesis; for treatment of a subject in need of treatment with a neurogenic compound; and/or for treatment of a disease or condition associated with damage to the hippocampus.

As used herein, the term "neurogenic compound" refers to a compound that exhibits neurogenic properties, *i.e*., stimulates growth of neurons *in vitro* and/or *in vivo.* For purposes of this definition, such effects can be measured as is known in the art, for example, as described in the Examples, e.g. *in vitro* as described in Shah et al., Quantitation of neurite growth parameters in explant cultures using a new image processing program, J. Neurosci. Methods, 136(2):123-31 (2004) and Ronn et al., A simple procedure for quantification of neurite outgrowth based on stereological principles, J. Neurosci. Methods, 100(1-2):25-32 (2000), each hereby incorporated herein by reference, or *in vivo* using magnetic resonance imaging of hippocampal volume and/or measuring changes in cerebral blood volume.

The present invention relates to a compound, and a composition comprising an effective amount of the compound, having the following formula I, a pharmaceutically acceptable salt thereof, or a metabolite thereof: 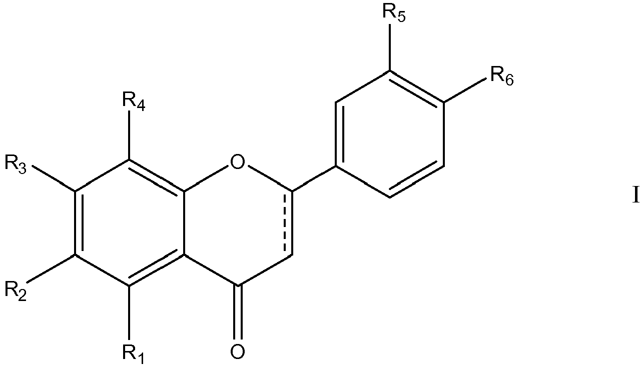 wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose.

In certain embodiments, the invention relates to the compound of formula I, wherein R₁ and/or R₃ is hydroxyl.

For example, the present invention also relates to a compound, and a composition comprising an effective amount of the compound, having the following formula I, a pharmaceutically acceptable salt thereof, or a metabolite thereof: 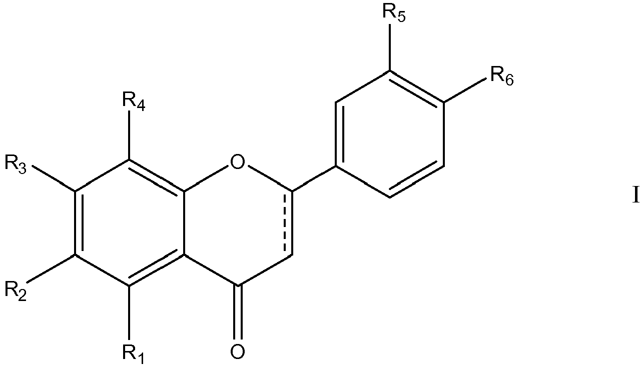 wherein
R₁, R₃, and R₅ are each independently selected from the group consisting of hydrogen,
-OCH₃, O-CH₂-CH₃, O-gallate and O-sugar, wherein the sugar is selected from the group consisting of glucose, maltose, gallactose and fructose;
wherein
R₂ and R₄ are each independently selected from the group consisting of hydrogen,
hydroxyl, -OCH₃, O-CH₂-CH₃, O-gallate and O-sugar, wherein the sugar is selected from the group consisting of glucose, maltose, gallactose and fructose; and
wherein
R₆ is selected from the group consisting of O-CH₂-CH₃, O-gallate and O-sugar,
wherein the sugar is selected from the group consisting of glucose, maltose, gallactose and fructose.

Examples of the compounds of formula I include apigenin, luteolin, baicalein, amentoflavone, kaempferol, chrysin, chryseriol, diosmetin, isorhamnetin and acacetin. In certain embodiments, the invention relates to the compound of formula I, wherein R₁ and/or R₃ is hydroxyl.

Metabolites of the above compounds of formula I may be glycosylated (e.g. glucuronidated), sulfated, and/or methylated metabolites. For example, glycosylation may occur at carbon positions C7 and/or C4', e.g. apigenin-7-O-glucuronide (apigenin-7-O-beta glucuronide), apigenin-7-O-glucoside, luteolin-7-O-glucoside, apigenin-4'-O-glucuronide (apigenin-7-O-beta glucuronide), apigenin 4'-O-sulfate-7-O-glucuronide.

Also within the scope of the invention are dimers of the above formula I such that two monomers of formula I are bonded via a 8→3' bond.

In other embodiments, the invention relates to a compound, and a composition comprising an effective amount of the compound, having the following formula II, a pharmaceutically acceptable salt thereof, or a metabolite thereof: 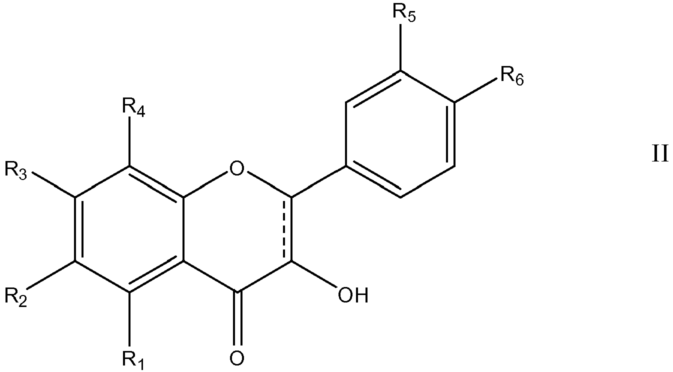 wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose.

Examples of the compounds of formula II include geraldol, quercetin and fisetin hydrate. In certain embodiments, the invention relates to the compound of formula II, wherein R₁ and/or R₃ is hydroxyl; in yet other embodiments, the invention relates to the compound of formula II, wherein R₅ and/or R₆ is hydroxyl.

Metabolites of the above compounds of formula II may be glycosylated (e.g. glucuronidated,), sulfated, and/or methylated metabolites. For example, quercetin-3-O-glucuronide (quercetin-3-O-beta glucuronide), quercetin-3, 4'-di-glucoside, quercetin-4'-O-glucoside, quercetin-3-glucoside, quercetin-3-sulfate, quercetin glucoside sulfate, 3'-methylquercetin 3-O-glucuronide.

Further within the scope of the invention is a compound, and a composition comprising an effective amount of the compound, having the following formula III, a pharmaceutically acceptable salt thereof, or a metabolite thereof: 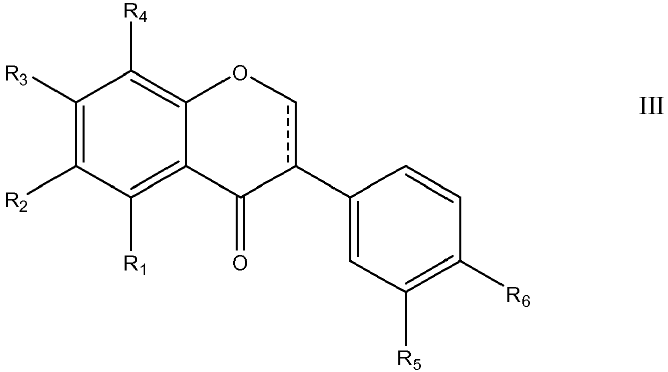 wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose.

An example of the compounds of formula III is genistein. In certain embodiments, the invention relates to the compound of formula III, wherein R₁ and/or R₃ is hydroxyl.

Metabolites of the above compounds of formula III may be glycosylated (e.g. glucuronidated), sulfated, and/or methylated metabolites. For example, genistein sulfate (e.g. genistein-7-sulfate), genistein glucuronide (e.g. genistein-7-O-glucuronide, genistein-4'-O-glucuronide), genistein sulfoglucuronide.

In other embodiments, the invention relates to a compound, and a composition comprising an effective amount of the compound, having the following formula IV, a pharmaceutically acceptable salt thereof, or a metabolite thereof: 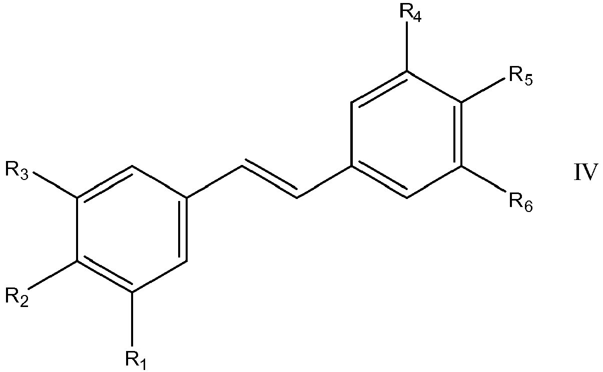 wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose.

An example of the compounds of formula IV includes resveratrol. In certain embodiments, the invention relates to the compound of formula IV, wherein R₂, R₄ and R₆ are hydroxyl.

In further embodiments, the invention relates to a compound, and a composition comprising an effective amount of the compound, having the following formula V, a pharmaceutically acceptable salt thereof, or a metabolite thereof: 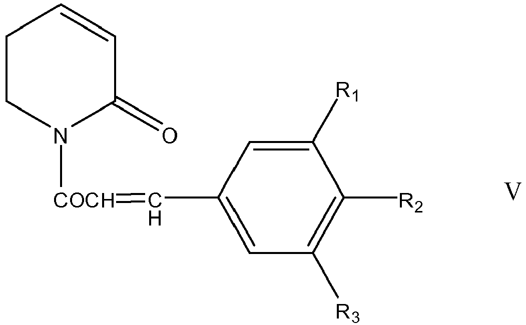 wherein
R₁, R₂, and R₃ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose.

An example of the compounds of formula V includes piperlongumine. In certain embodiments, the invention relates to the compound of formula V, wherein R₁, R₂ and R₃ are -OCH₃.

In yet further embodiments, the invention relates to a compound, and a composition comprising an effective amount of the compound, having the following formula VI, a pharmaceutically acceptable salt thereof, or a metabolite thereof: 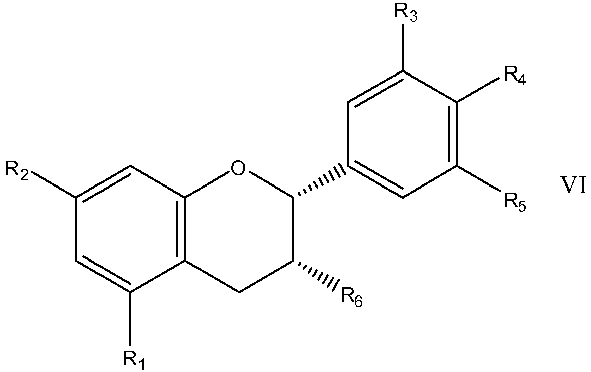 wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose.

In certain embodiments, the invention relates to a compound, and a composition comprising an effective amount of the compound, having the following formula VI, a pharmaceutically acceptable salt thereof, or a metabolite thereof: 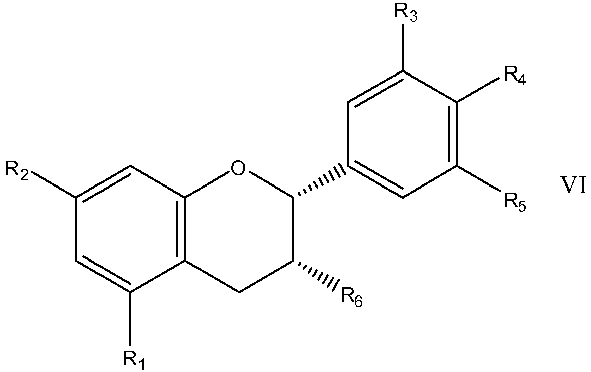 wherein
R₁, R₂ and R₆ are each independently selected from the group consisting of hydrogen, -OCH₃, O-CH₂-CH₃, O-gallate and O-sugar, wherein the sugar is selected from the group consisting of glucose, maltose, gallactose and fructose, and
wherein
R₃, R₄ and R₅ are each independently selected from the group consisting of hydrogen, O-CH₂-CH₃, O-gallate and O-sugar, wherein the sugar is selected from the group consisting of glucose, maltose, gallactose and fructose.

Examples of the compounds of formula VI include (-)-epicatechin gallate and (-)-epigallocatechin gallate. In certain embodiments, the invention relates to the compound of formula VI, wherein R₁, R₂, R₃, R₄ and/or R₅ are hydroxyl and R₆ is O-gallate.

The compound of the invention may be prepared as is known to a person of skill in the art. For example, they may be obtained from natural sources such as chamomile and parsley (apigenin), onion (quercetin), broccoli and artichokes (luteolin), or Chinese medicinal herbs (baicalein). See, for example, Avallone et al., Pharmacological profile of apigenin, a flavonoid isolated from Matricaria chamomilla, Biochem. Pharmacol. 59(11):1357-94 (2000); Janssen et al., Effects of the flavonoids quercetin and apigenin on homeostasis in healthy volunteers, Am. J. Clin. Nutr. 67(2):255-62 (1998); Miean et al., Flavonoid (myricetin, quercetin, kaempferol, luteolin, and apigenin) content of edible tropical plants, J. Agric. Food Chem. 49(6):3106-12 (2001); Brown et al., Luteolin-rich artichoke extract protects low density lipoprotein from oxidation in vitro, Free Radic. Res., 29(3):247-55 (1998); Suk et al., Flavonoid baicalein attenuates activation-induced cell death of brain microglia, J. Pharmacol. Exp. Ther. 305(2):638-45 (2003), each hereby incorporated herein by reference. The compounds are also commercially available for purchase, for example through Sigma, Aldrich, and InterContinental Chemicals (ICC).

The compound may be "isolated and purified," *i.e.,* it may be separated from the compounds with which it naturally occurs (e.g. when the compound is of natural origin), or it may be "synthetically prepared" (*i.e.,* manufactured using a process of synthesis) in either case such that the level of contaminating compounds and/or impurities does not significantly contribute to, or detract from, the effectiveness of the compound. Such compounds are particularly suitable for pharmaceutical applications. In some embodiments, the compound of the invention is at least 80% pure, at least 85% pure, at least 90% pure, at least 95% pure, at least 98% pure, or at least 99% pure. Such compounds are particularly suitable for pharmaceutical applications. Use of less pure plant extracts containing the compounds described herein is also within the scope of the invention; such extracts may be suitable for food and dietary supplement applications.

### Methods of Use

Any compound and/or composition described herein may be used to practice the methods described in the present application.

The invention relates to method(s) of use of the compound(s) described herein, e.g. method for stimulating neurogenesis, including *in vitro* neurogenesis, by contacting neuronal progenitor cells with an effective amount of the compound(s) described herein; method for treatment of a subject in need of treatment with a neurogenic compound; and/or for treatment of a disease or condition associated with damage to the hippocampus. The subject may be a human or a veterinary animal.

A person of skill in the art will be able to envision subjects that can benefit from the methods of treatment described herein. For example, such subjects include, but are not limited to, a subject with damage to the hippocampus; a subject suffering from, or at risk of, oxygen starvation and/or deprivation; a subject suffering from encephalitis; a subject suffering from epilepsy, a subject suffering from a mood disorder (*e.g*. depression or post-traumatic stress disorder); a subject suffering from memory function impairment (*i.e.* an inability to form new memories about personally experienced events, *e.g.,* a subject having problems with episodic memory, and/or with access to memories prior to the damage, such as a subject suffering from anterograde amnesia or retrograde amnesia); a subject in need of increasing hippocampus-associated learning, a subject suffering from navigation impairment (*e.g*, disorientation, subjects failing to remember places they have been to and how to get where they are going); a subject having a profession which requires navigation; a subject suffering from, or at risk of, sleep deprivation (*e.g*. a subject having a profession that involves sleep deprivation, a soldier or combatant), a subject exposed to stress (*e.g*. prolonged stress, high level of stress, a soldier or combatant); a subject exposed to physical and/or mental exertion (*e.g*. soldier or combatant), an elderly subject (*e.g.,* a healthy elderly subject such as a subject not diagnosed with a neurodegenerative, e.g. Alzheimer's disease); a subject with decreased/low levels of serotonin; a subject taking medication that decreases/affects hippocampal neurogenesis, a subject taking amphetamines (*e.g*. methyphenidate); a subject taking opiates; a subject experiencing elevated levels of glucocorticoids. In some embodiments, the subject suffers, or is at risk of suffering of, any of the above conditions other than a neurodegenerative disease. With respect to subjects suffering from neurodegenerative diseases, it will be understood that the compounds described herein are not used to treat the condition itself, therefore only certain subsets of such patients may benefit from the methods described herein. Such subjects would be apparent to persons of skill in the art, *e.g*. a subject suffering from an early stage Alzheimer's disease.

As used herein, the term "elderly subject" refers to a subject who is sixty-five and greater years of age. As used herein, "healthy elderly subject" refers to an elderly subject who is not suffering from and/or has not been clinically diagnosed with a neurodegenerative disease.

As used herein, "treatment" means improving an existing condition, for example by slowing down a disease progression, prolonging survival, reducing the risk of death, providing a measurable improvement of disease parameters and/or slowing down natural deterioration in the elderly. With respect to cognitive impairment, "treatment" refers to a measurable improvement of at least one of the following: disorientation, memory dysfunction (including episodic memory, autobiographical memory, declarative memory, anterograde amnesia, and retrograde amnesia), of symptoms or adverse effects of sleep deprivation, and depression. As used herein, "treatment of depression" means improving at least one of the symptoms of depression.

As used herein, "exercise" refers to an activity requiring physical exertion by a human or veterinary animal.

Thus, the invention relates to a method for stimulating neurogenesis (*in vivo* and/or *in vitro*); a method of treating a subject in need of treatment with a neurogenic compound; and/or a method of treating a disease or condition associated with damage to the hippocampus by administering to a subject in need thereof, either alone or in combination with physical exertion (e.g. exercise), an effective amount of a compound of the following formula I, a pharmaceutically acceptable salt thereof, or a metabolite thereof: 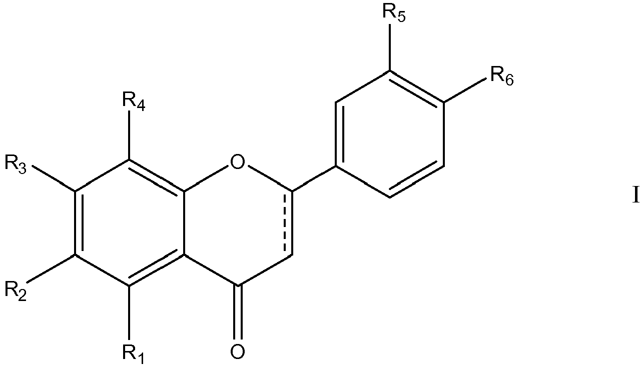 wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose.

For example, the present invention relates to a compound, and a composition comprising an effective amount of the compound, having the following formula I, a pharmaceutically acceptable salt thereof, or a metabolite thereof: 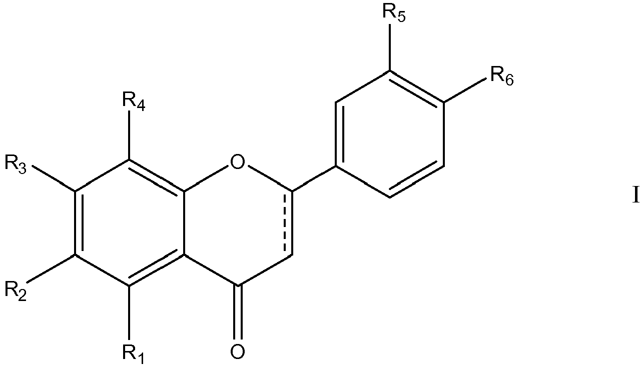 wherein
R₁, R₃, and R₅ are each independently selected from the group consisting of hydrogen, -OCH₃, O-CH₂-CH₃, O-gallate and O-sugar, wherein the sugar is selected from the group consisting of glucose, maltose, gallactose and fructose,
wherein
R₂ and R₄ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose, and
wherein
R₆ is selected from the group consisting of O-CH₂-CH₃, O-gallate and O-sugar, wherein the sugar is selected from the group consisting of glucose, maltose, gallactose and fructose.

In other embodiments, the above methods are practiced by administering the compound of the following formula II, a pharmaceutically acceptable salt thereof, or a metabolite thereof: 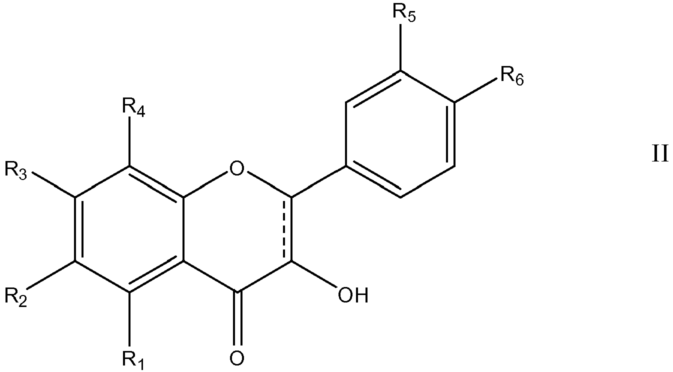 wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose.

In further embodiments, the above methods are practiced by administering the compound of the following formula III, a pharmaceutically acceptable salt thereof, or a metabolite thereof: 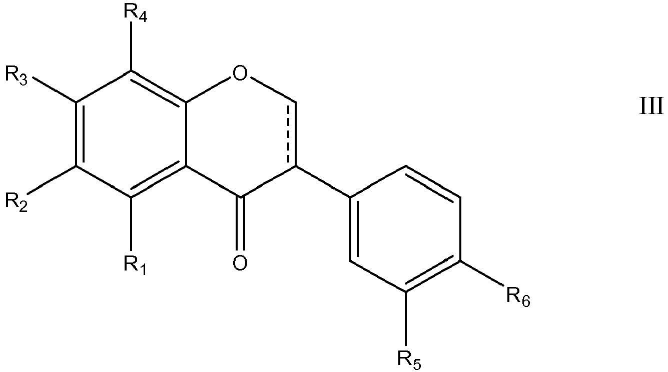 wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose.

In yet further embodiments, the above methods are practiced by administering the compound of the following formula IV, a pharmaceutically acceptable salt thereof, or a metabolite thereof: 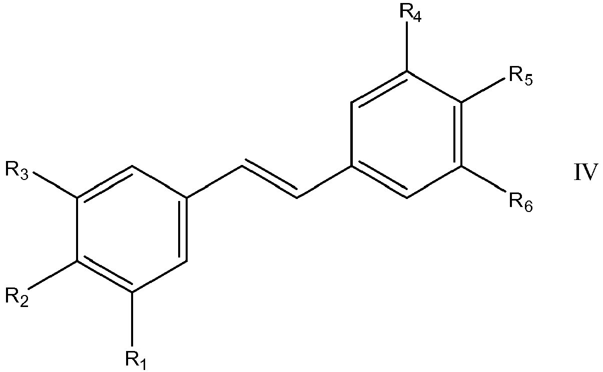 wherein
R₁, R₂, R₃, R₄, and R₅ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose.

In yet further embodiments, the above methods are practiced by administering the compound of the following formula V, a pharmaceutically acceptable salt thereof, or a metabolite thereof: 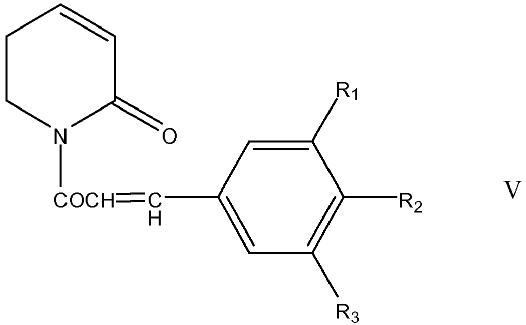 wherein
R₁, R₂, and R₃ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose.

In yet further embodiments, the above methods are practiced by administering the compound of the following formula V, a pharmaceutically acceptable salt thereof, or a metabolite thereof: 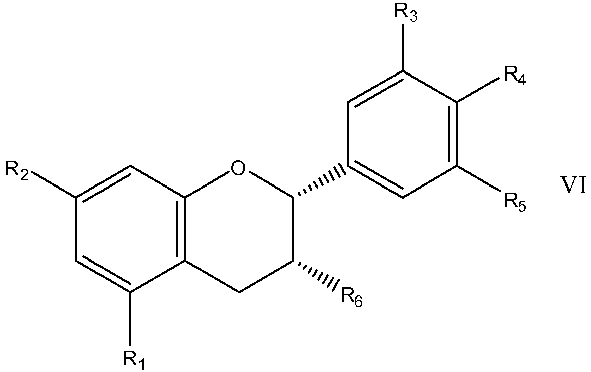 wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose.

For example, the invention relates to a compound, and a composition comprising an effective amount of the compound, having the following formula VI, a pharmaceutically acceptable salt thereof, or a metabolite thereof: 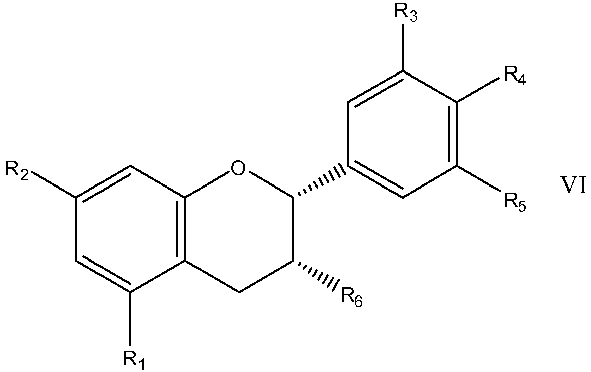 wherein
R₁, R₂ and R₆ are each independently selected from the group consisting of hydrogen, -OCH₃, O-CH₂-CH₃, O-gallate and O-sugar, wherein the sugar is selected from the group consisting of glucose, maltose, gallactose and fructose, and
wherein
R₃, R₄ and R₅ are each independently selected from the group consisting of hydrogen, O-CH₂-CH₃, O-gallate and O-sugar, wherein the sugar is selected from the group consisting of glucose, maltose, gallactose and fructose.

Examples of the compounds of formulas I-VI, and their metabolites for use in the methods described herein are as described above.

The present compounds may be used in combination therapy with other compounds suitable for use for treatment of diseases or conditions recited herein. Examples of such agents will be apparent to persons of skill in the art; for example, cannabinoids, anti-depressants (for example, fluoxetine), brain-derived neurotropic factor (BDNF), or insulin-like growth factor (IGF-1).

As noted above, the methods of the invention may be used in a human or a veterinary animal. As used herein, "veterinary animal" refers to any animal cared for, or attended to by, a veterinarian, including companion (pet) animals and livestock animals, such as a dog, a cat, and a horse.

The effective amount for use in the above methods may be determined by a person of skill in the art using the guidance provided herein and general knowledge in the art.

The compounds of the invention may be administered at from about 1 mg/day to about 2000 mg/day, preferably from about 100 mg/day to about 1000 mg/day, and most preferably from about 250 mg/day to about 500 mg/day. However, amounts higher than stated above may be used.

The compounds may be administered acutely, or treatment administration may be continued as a regimen, *i.e*., for an effective period of time, e.g., daily, monthly, bimonthly, biannually, annually, or in some other regimen, as determined by the skilled medical practitioner for such time as is necessary. The administration may be continued for at least a period of time required to exhibit therapeutic effects. Preferably, the composition is administered daily, most preferably two or three times a day, for example, morning and evening to maintain the levels of the effective compound in the body of the mammal. To obtain the most beneficial results, the composition may be administered for at least about 30, or at least about 60 days. These regimens may be repeated periodically. Based on the guidance provided herein and general knowledge in the art, a person of skill in the art can select compounds that are suitable for acute and or/chronic administration. Thus, dosage forms adapted for such administration (e.g. acute, chronic) are within the scope of the invention.

A physical exercise routine has been shown to increase metabolic rate, cellular respiration, and blood flow sufficient to enhance neurogenesis and cognitive performance. The combination of physical exercise with the administration of the inventive compounds is within the scope of this invention.

Also within the scope of the invention are assays for determining a minimum therapeutically required dosage amount or an optimal dosage amount for use in the above therapeutic methods. Methods described in the examples, or any other dose response methods known to be predictive of compound effectiveness to treat the subject(s) recited herein may be used. Compounds described herein may be tested in such assays. Dosage forms adapted to deliver at least a minimum therapeutically effective amount, or an optimal amount, are within the scope of the invention.

### Compositions and Formulations

The compounds of the invention may be administered as a pharmaceutical, food, food additive or a dietary supplement.

As used herein a "pharmaceutical" is a medicinal drug. See Merriam-Webster's Collegiate Dictionary, 10th Edition, 1993. A pharmaceutical may also be referred to as a medicament. A "food" is a material containing protein, carbohydrate and/or fat, which is used in the body of an organism to sustain growth, repair and vital processes and to furnish energy. Foods may also contain supplementary substances: for example, minerals, vitamins and condiments. See Merriam-Webster's Collegiate Dictionary, 10th Edition, 1993. The term food includes a beverage adapted for human or animal consumption. A "food additive" is as defined by the FDA in 21 C.F.R. 170.3(e)(1) and includes direct and indirect additives. A "dietary supplement" is a product (other than tobacco) that is intended to supplement the diet and contains the one or more of the following dietary ingredients: a vitamin, a mineral, an herb or other botanical, an amino acid, a dietary substance for use by man to supplement the diet by increasing the total daily intake, or a concentrate, metabolite, constituent, extract or combination of these ingredients. As used herein, a "dietary composition" includes food, dietary supplement and/or food additive. Such compositions may be prepared as is known in the art.

Pharmaceuticals containing the inventive compounds, optionally in combination with another compound (e.g. neurogenic compound), may be administered in a variety of ways such as orally, sublingually, bucally, nasally, rectally, intravenously, parenterally and topically. A person of skill in the art will be able to determine a suitable mode of administration to maximize the delivery of the compound of the invention and optionally a vascular-protective agent. Thus, dosage forms adapted for each type of administration are within the scope of the invention and include solid, liquid and semi-solid dosage forms, such as tablets, capsules, gelatin capsules (gelcaps), bulk or unit dose powders or granules, emulsions, suspensions, pastes, creams, gels, foams or jellies. Sustained-release dosage forms are also within the scope of the invention. Suitable pharmaceutically acceptable carriers, diluents, or excipients are generally known in the art and can be determined readily by a person skilled in the art. The tablet, for example, may comprise an effective amount of the compound of the invention and optionally a carrier, such as sorbitol, lactose, cellulose, or dicalcium phosphate.

The foods comprising the compounds described herein, and optionally another neuro-protective agent may be adapted for human or veterinary use, and include pet foods. A daily effective amount of the compound of the invention may be provided in a single serving (in case of food) or a single dosage form (in case of a pharmaceutical or dietary supplement).

Further within the scope of the invention is an article of manufacture such as a packaged product comprising the composition of the invention (e.g. a food, a dietary supplement, a pharmaceutical) and a label indicating the presence of, or an enhanced content of the inventive compounds and/or directing use of the composition for methods described herein. An article of manufacture (such as a packaged product or kit) adapted for use in combination therapy comprising at least one container and at least one compound of the invention, or a pharmaceutically acceptable salt thereof is also provided. The article of manufacture may further comprise at least one additional agent, a neurogenic or therapeutic agent (*i.e*., other than the compound of the invention, or a pharmaceutically acceptable salt thereof), which agent may be provided in a separate container, or in admixture with the compound of the invention.

The invention also relates to methods of manufacturing an article of manufacture comprising any of the compositions described herein, packaging the composition to obtain an article of manufacture and instructing, directing or promoting the use of the composition/article of manufacture for any of the uses described herein. Such instructing, directing or promoting includes advertising.

The invention is further described in the following non-limiting examples.

### Example 1: Long-Term Injection Study of Apigenin

A six-week study was conducted in which apigenin was administered by injection to mice at two different doses in conjunction with or without daily voluntary exercise (running). Behavioral testing was administered after 30 days of injections using the Morris water maze and object recognition test.

### Materials and Methods

Apigenin was dissolved in [10:90] Tween 80: 0.9% NaCl. All compounds were purchased from commercial sources. Aliquots of Tween 80: 0.9% NaCl solutions were used for testing in the *in vivo* model.

Sixty-four mice (age seven wks) were divided into four test groups (sixteen per group) and received either:
0.9% NaCl,
Tween 80/0.9% NaCl,
Apigenin 12.5mg/kg, or
Apigenin 25mg/kg

Injections were administered intraperitoneally (i.p.) for forty-two days total. In addition to test injections, daily i.p. injections of 5-bromo-2-deoxyuridine (BrdU) [10mg/ml] were administered for the first eight days at a dose of 50mg/kg.

Within each test group, eight mice were randomly selected to receive running exercise. These thirty-two mice (eight mice per injection group) were transferred daily (on Days 1-40 of the study) from their normal static housing to an automated voluntary running wheel cage for two hours a day. Non-running mice remained in their static cages throughout the study.

On Day 31, all mice began acquisition training on the Morris water maze immediately following their i.p. injection. *See, e.g.,* Morris, Developments of a water-maze procedure for studying spatial learning in the rat, J. Neurosci. Methods, 11(1) (1984), hereby incorporated herein by reference. Mice were trained for nine consecutive days, receiving two trials a day using a hidden platform paradigm. The platform was located in the northwest (NW) quadrant of the water maze. To measure the effect of apigenin (with or without exercise) on learning, latency to platform was recorded for each trial. After daily training on the water maze, non-running mice were returned to their normal static housing while running mice were placed into their running wheel cages for two hours. On Day 9 of the water maze training, the mice received a probe trial 4 hours after the last training. During the probe trial the platform was removed and retention of memory of the target NW quadrant was recorded.

### Results

During the acquisition (training) phase of the water maze testing, latency to platform was recorded. Statistical analysis revealed no behavioral differences between saline and Tween 80 test groups. Therefore, the data for these groups were combined into one control group for both the running and non-running condition. A one-way ANOVA test showed significance among groups (F (5,58) =2.915 p< .05). Specific comparisons using Fisher's PLSD showed that both the apigenin 12.5mg/kg-runners and apigenin 25mg/kg-runners had significantly shorter latencies in finding the platform than their respective control runners and the control non-runners (p< .05). In addition, specific comparisons revealed that the apigenin 25mg/kg non-runner group was significantly different from the control runners (p< .05). No significant differences were found between the apigenin 12.5mg/kg non-runners and the control.

During probe trial testing, both frequency into target quadrant and total duration of time spent in target quadrant were measured (Figure 1A-D). Neither the sedentary nor the running controls showed a preference for the target quadrant. However, apigenin 12.5mg/kg-runners showed a significant preference for the target quadrant in the measure of frequency (p< .001) (Figure 1A). It can also be seen in Figure 1A that apigenin 25mg/kg-runners had a strong preference for entering the target quadrant more frequently than other quadrants, although this is not statistically significant (p< .06). Moreover, apigenin 25mg/kg non-runners showed a significant preference for the target quadrant in the measure of frequency (p< .05) (Figure 1B). Although not significant, apigenin 12.5mg/kg non-runners showed a trend toward significance (p< .06) for the target quadrant in the measure of frequency as well (Figure 1B). Analysis of total duration of time spent in the quadrants during the probe found no significant differences among the groups (Figures 1C and 1D).

In summary, apigenin increases learning with or without exercise, although exercise provided an additional benefit at lower apigenin doses.

### Example 2: Object Recognition Study

### Materials and Methods

Each mouse receiving either apigenin 25mg/kg or Tween 80:0.9% NaCl (control) solutions was administered an i.p. injection of either drug or control for 7 days, after which they were trained and tested on visual paired comparison task (VPC) behavioral assay. The paradigm consisted of habituation to the experimental room for two hours prior to the experiment on Days 1 and 2. After habituation on Day 1, mice were given a five minute exposure to the testing chamber, followed by a thirty second exploration of two identical objects (familiarization training). Twenty-four hours after the familiarization training, mice were once again re-habituated to the room and testing chamber for two hours and one minute, respectively. After re-habituation mice were placed back into the testing chamber and allowed to explore a familiar and novel object for five minutes. Object exploration times were measured for both familiar and novel objects on Days 1 and 2. Both objects were identical, but the "novel" object was in a different location.

### Results

Both groups learned the object recognition paradigm and spent more time exploring the novel object on Day 2 than the familiar object from Day 1. T-test analysis revealed that both the control and apigenin groups showed a significant preference for the novel object (t=0.03 and t=.0008, respectively). No significant differences were seen between injection groups during novel testing.

### Example 3: Histological Analysis of Brain Tissue

After the long-term study of Example 1 and all behavioral testing was concluded (Day 42), mice were perfused using 4% paraformaldehyde and their brain tissue was extracted. Brain tissue was sectioned and stained for BrdU/DAB quantification of dividing cells. A second immunofluorescence stain was used for phenotyping of the newly dividing cells. Brain sections were also taken and stained with Brdu/NeuN/GFAP. Phenotype analysis was completed to determine the percentage of dividing cells co-labeled with NeuN, a marker used to identify mature neurons. Using the percentage, it was possible to calculate the total number of BrdU/NeuN co-labeled cells in the dentate gyrus.

### Results

Quantification of the BrdU/DAB stained tissue showed a significant increase in dividing cells between the running mice and non-running mice (F _{(5,34)} = 16.855 p ≤ .0001) (Fig. 2). A significant increase in the number of dividing cells for the apigenin 25mg/kg non-runners when compared to both control non-runners (p <.01) and to apigenin 12.5mg/kg non-runners (p <.01). Apigenin 25mg/kg-runners also had significantly more dividing cells (p < .05) when compared to their running control (Figure 2).

Regarding BrdU/NeuN/GFAP staining, statistical analysis revealed a significant increase in neurogenesis among groups (F_{(5, 34)}= 20.9 p<.0001) (Figure 3). Post-hoc analysis also reveals that running mice showed a significant increase in neurogenesis in comparison to non-running mice (p<.01). More importantly, apigenin 25mg/kg runners had a significantly higher number of new neurons than all other mice in the study (p<.001).

The data shows that apigenin increases both the amount of dividing cells in the dentate gyrus and the differentiation of the dividing cells into neurons. Running in combination with apigenin produced a further increase of neurogenesis. Apigenin 25mg/kg runners had significantly more new neurons than all other groups. When compared with the results of the water maze test (Example 1), the best performers were those mice that had the most neurogenesis.

### Example 4: Comprehensive Apigenin Study (Oral and I.P. Administration)

A 10-day food study was conducted in which the effects of oral administration of apigenin were compared with apigenin effects upon injection. Food pellets containing epicatechin were tested alongside apigenin.

### Materials and Methods

Animals were fed food containing apigenin, epicatechin or control pellets in two doses: 250ppm and 500ppm.

Aliquots of apigenin and epicatechin were dissolved in [10:90] Tween 80: 0.9% NaCl. Aliquots of Tween 80: 0.9% NaCl were used as controls.

Forty mice (age seven weeks) were divided into three test injection groups (n=five per group) and five test food groups (n=five per group) and received:
Tween 80:0.9% NaCl by injection,
Apigenin 25mg/kg by injection,
Epicatechin 20mg/kg by injection,
Control Food,
Apigenin Food 250ppm,
Apigenin Food 500ppm,
Epicatechin Food 250ppm, or
Epicatechin Food 500ppm

Test injections were administered i.p. for ten days total. Test food was distributed on Day 1 and mice were allowed to eat freely for the duration of the ten days. Daily i.p. injections of BrdU [10mg/ml] were administered to all animals the first eight days of this study at a dose of 50mg/kg.

At the end of ten days, all mice were perfused using 4% paraformaldehyde and their brain tissue was extracted. Brain tissue was sectioned and stained for BrdU/DAB quantification for dividing cells.

### Results

Quantification of the BrdU/DAB stained tissue revealed a significant increase in the number of dividing cells in the dentate gyrus of the mice receiving apigenin regardless of the administration route (p< .01) (Figure 4). No significant differences were found between epicatechin and controls.

### Example 5: The effect of basic fibroblast growth factor (FGF2) on Apigeinin Induced Neurogenesis

### Materials and Methods

Adult neural progenitor cells were plated in N2 media containing FGF2 for twenty-four hours. *See* Bottenstein et al., Growth of a rat neuroblastoma cell line in serum-free supplemented medium, Proc. Natl. Acad. Sci. USA 76(1) (1979) (describing components of N2 media).

The cells were replaced with fresh N2 media (without FGF2) and apigenin was added. In all experiments, 5mM apigenin dissolved in DMSO was used. As a positive control for neuronal differentiation, N2 media containing retinoic acid (RA) and forskolin (FSK) was used. As a negative control, N2 media and the equivalent amount of DMSO or N2 was used. Either a NeuroD luciferase reporter assay (purchased from Promega, cat. No. e-1980, Dual-Luciferase Reporter 1000 Assay Sytem) was performed two days later or immunohistochemistry was performed four days later.

The immunohistochemical analysis of the apigenin-treated and control cells was performed by fixing and staining the cells after four days with lineage-specific markers (Tuj 1 for neurons, GFAP for astrocytes, and RIP for oligodendrocytes). Tuj 1 is a neuron-specific class III beta-tubulin used as a marker for new neurons. GFAP (glial fibrillary acidic protein), an intermediate-filament protein which is expressed almost exclusively in the astrocytes of the central nervous system, is used as a marker for new astrocytes. RIP is a monoclonal antibody used as an early marker of developing oligodendrocytes.

### Results

NeuroD luciferase results are reported in Figure 5. There was a two-fold higher NeuroD-activation by apigenin as compared to activation by RA + FSK when cells are first allowed to proliferate with FGF2 for twenty-four hours.

Immunohistochemistry results are reported in Figure 6. There was a higher percentage of Tuj1-positive neurons (30-60%) in RA+FSK-treated cells than in apigenin-treated cells when cells were allowed to first proliferated with FGF2 for twenty-four hours (positive control). Interestingly, there are more Tuj 1-positive neurons (from 5 to 30%) in apigenin-treated cells when they are directly plated into N2 media without FGF2. Although this is unexpected, considering the effect of apigenin on NeuroD-luciferase and RA+FSK, the experiment was repeated a number of times and no significant difference in Tuj 1 or Map2ab-positive neurons in apigenin-treated cells with or without FGF-2 pre-treatment was ever observed. Apigenin's effects on astrocyte (GFAP) and oligodendrocyte (RIP) differentiation appear to be low (1-5%) when cells are first proliferating in FGF2. This suggests that the major role of apigenin in adult neural progenitor cells is to induce neuronal differentiation.

### Example 6: Western Blot Analysis of Apigenin Signaling Specificity

A study was conducted to (a) investigate downstream signaling mediators of apigenin and (b) determine the independent contribution of apigenin on neural progenitor proliferation, differentiation, and survival.

### Materials and Methods

Adult neural progenitor cells were grown in proliferating conditions (N2 containing FGF2) in six-well plates for at least twenty-four hours. Before collecting cell lysates, the media was removed and replaced with DMEM/F12 for two hours (DMEM/F12 is defined media which lacks insulin and FGF2, two factors known to trigger various cell signaling pathways). In this key "starvation" step, the activation of various downstream mediators returned to steady-state levels.

Cells were starved for two hours and the time zero data were collected. One of the following was added and the lysates collected at various time-points: (1) N2 only or (-) DMSO (negative control); (2) (+) DMSO (also a negative control); (3) apigenin; or (4) RA+FSK (positive control). Two time-course experiments were then carried out. The first was an early time-course to measure the acute effects of apigenin at zero minutes, ten minutes, thirty minutes, two hours, six hours, and twenty-four hours. The second experiment was a late time-course to measure long-term effects of apigenin at zero, one, two, three, and four days; the results of this experiment are not discussed here.

Cell lysates were then loaded and transferred to Western Blots and the membranes hybridized with antibodies against the phosphorylated and unphosphorylated forms of each protein. The data results with the unphosphorylated form of the protein are not discussed here. The data discussed with respect to the phosphorylated protein are representative of at least three independent experiments and are normalized to the unphosphylated forms (internal control).

### Result

The activation status of various signaling mediators after apigenin treatment to the positive control (RA+FSK) and negative controls [(-) DMSO and (+) DMSO] were compared in order to determine which pathways were specific to the presence of apigenin. Five cellular pathways were chosen (Akt, ERKs, GSK3b, b-catenin, and CREB) based on either the literature or laboratory evidence that these pathways are involved in neurogenesis. These experiments demonstrate that apigenin has non-specific effects in the phosphorylation of ERKs, GSK3b, b-catenin, and CREB. Negative controls show the same results as cells treated with apigenin.

Interestingly, there does appear to be a specific effect of increased phosphorylation of AKT (by six hours) after apigenin treatment. This does not occur in the negative controls; decreased phosphorylation of AKT with DMSO treatment alone may occur. Furthermore, RA+FSK also appeared to increased phosphorylated-AKT (pAKT). These results suggest that 5mM apigenin specifically activates phosphorylated AKT (pAKT).

### Example 7: NeuroD Neurogenic Activity of Apigenin and Other Compounds Materials and Methods:

Adult neural progenitor cells were treated with different concentrations of the neurogenic compounds as described in Example 5, including the following: apigenin (5 µM) (purchased from Aldrich); acacetin (10 µM) (ICC); luteolin (98%) (.5 (1.0) µM) (ICC); baicalein (2 µM) (ICC); amentoflavone (2 µM) (ICC); genistein (99%+) (5 µM) (ICC); kaempferol (10 µM) (Sigma); taxifolin (10 µM) (ICC); geraldol (10 µM) (ICC); chrysoeriol (10 µM) (ICC); resveratrol (20 µM) (Sigma); (-) epicatechin gallate (5 µM) (Sigma); (-) epigallocatechin gallate (5 µM) (Sigma); and piperlongumine (97%) (5 µM) (ICC). The cells were harvested and assayed for luciferase activity as previously described in Example 5.

### Results

Of twenty-eight compounds tested during this reporting period, the fourteen compounds listed above showed an up-regulation of luciferase activity.

## Claims

1. A compound for use in treating a subject in need of treatment with a neurogenic compound, wherein the compound is selected from the group consisting of: a compound of formula I, a compound of formula II, a compound of formula III, a compound of formula IV, a compound of formula V, a compound of formula VI, a pharmaceutically acceptable salt of each, and a metabolite of each: 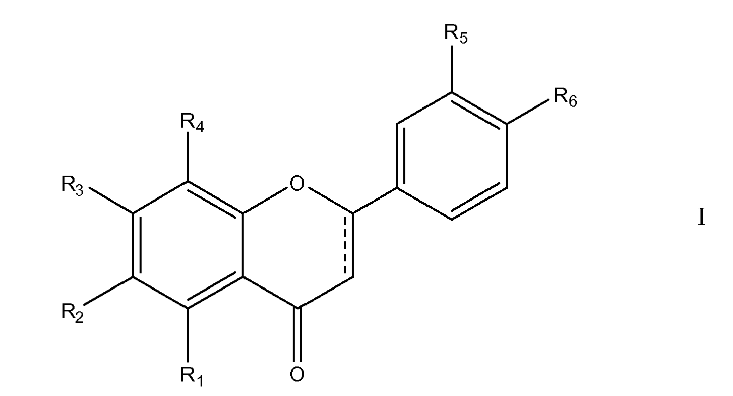 wherein
R¹, R², R³, R⁴, R⁵ and R⁶ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose; 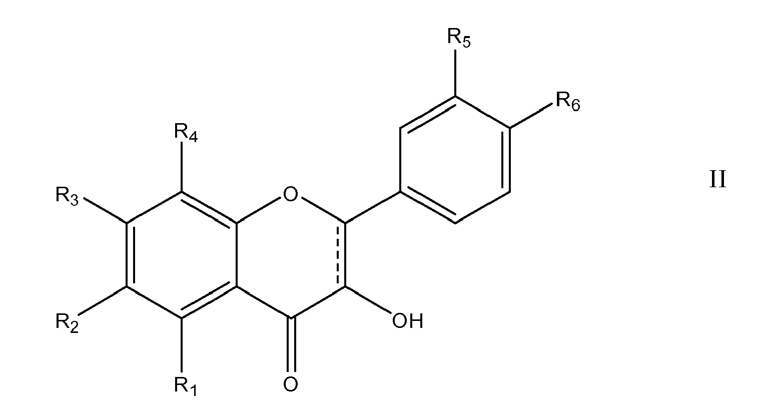
wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose; 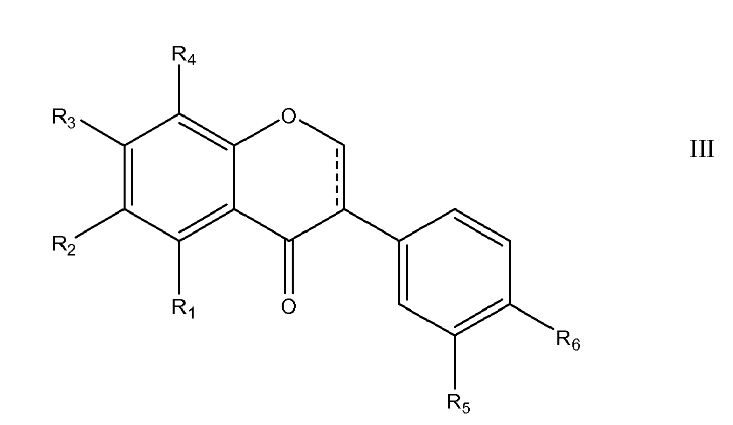
wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose; 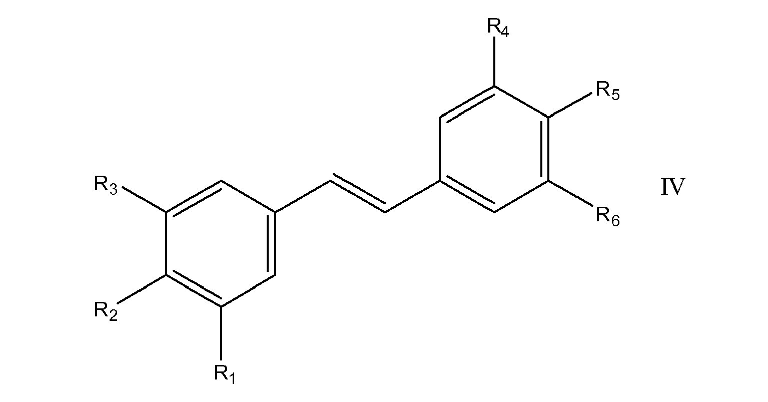
wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose; 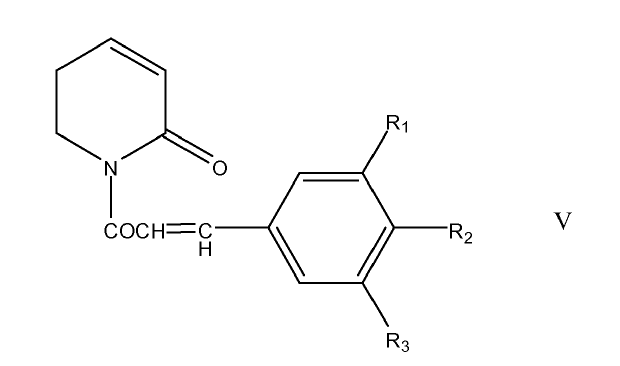
wherein
R₁, R₂, and R₃ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose; 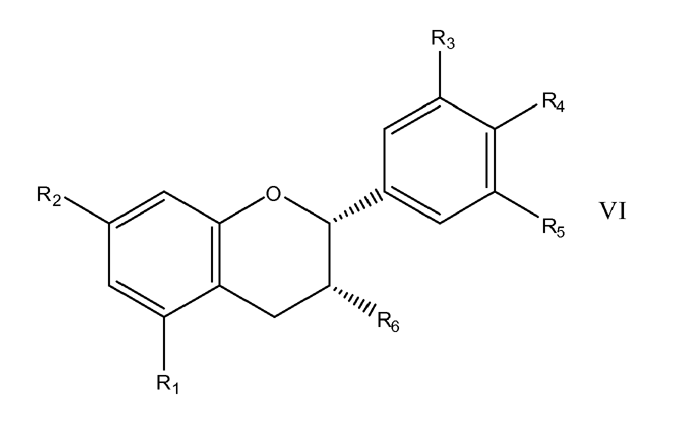
wherein
R₁, R₂ and R₆ are each independently selected from the group consisting of hydrogen, -OCH₃, O-CH₂-CH₃, O-gallate and O-sugar, wherein the sugar is selected from the group consisting of glucose, maltose, gallactose and fructose, and
wherein
R₃, R₄ and R₅ are each independently selected from the group consisting of hydrogen, O-CH₂-CH₃, O-gallate and O-sugar, wherein the sugar is selected from the group consisting of glucose, maltose, gallactose and fructose;
and wherein the subj ect is a human or a veterinary animal.

2. A compound for use according to claim 1, wherein the subject is a human.

3. A compound for use according to claim 2, wherein the compound is administered in combination with exercise.

4. A compound for use according to claim 2 , wherein the human is other than the one suffering from a neurodegenerative disease.

5. A compound for use according to claim 3, wherein the human is other than the one suffering from a neurodegenerative disease.

6. A compound for use according to claim 2, wherein the human is an elderly human.

7. A compound for use according to claim 3, wherein the human is an elderly human.

8. A compound for use according to claim 6, wherein the elderly human is a healthy elderly human.

9. A compound for use according to claim 7, wherein the elderly human is a healthy elderly human.

10. A compound for use according to claim 2, wherein the human suffers from an early stage Alzheimer's Disease.

11. A compound for use according to claim 2, wherein the human suffers from a disease or condition associated with damage to the hippocampus.

12. A compound for use according to claim 2, wherein the compound is administered without exercise.

13. A method of stimulating neurogenesis comprising contacting a neural progenitor cell with an effective amount of a compound selected from the group consisting of a compound of formula I, a compound of formula II, a compound of formula III, a compound of formula IV, a compound of formula V, a compound of formula VI, a pharmaceutically acceptable salt of each, and a metabolite of each: 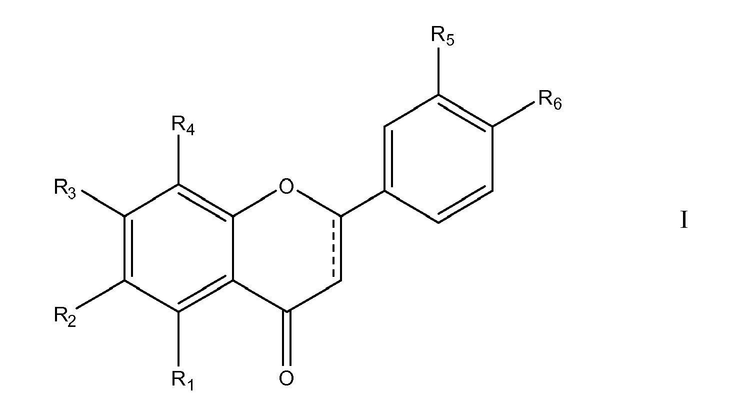 wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose; 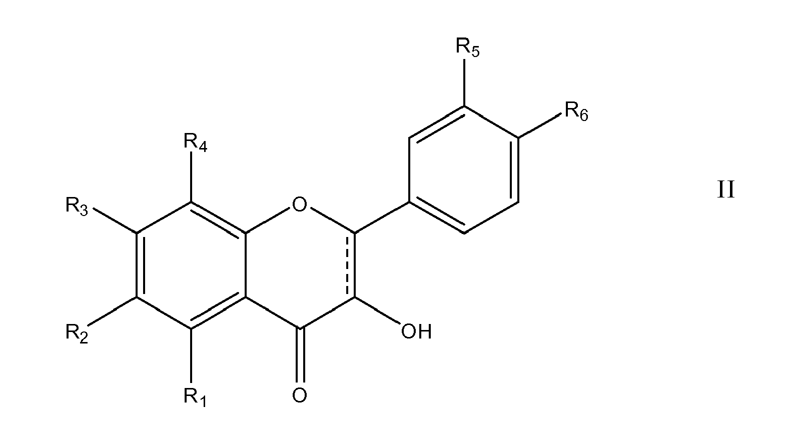
wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose; 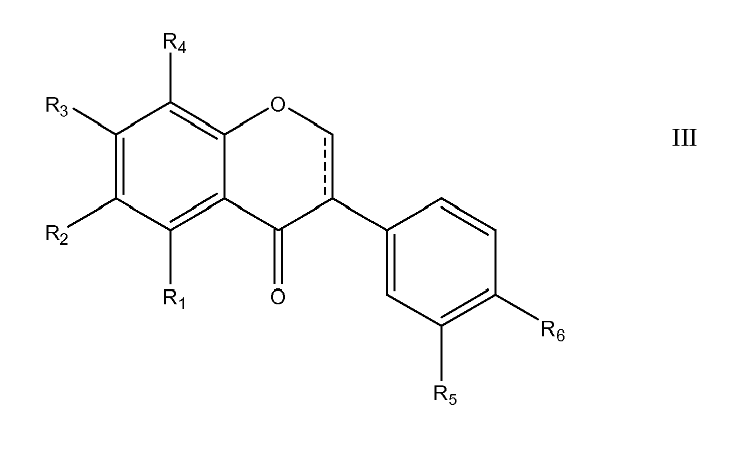
wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose; 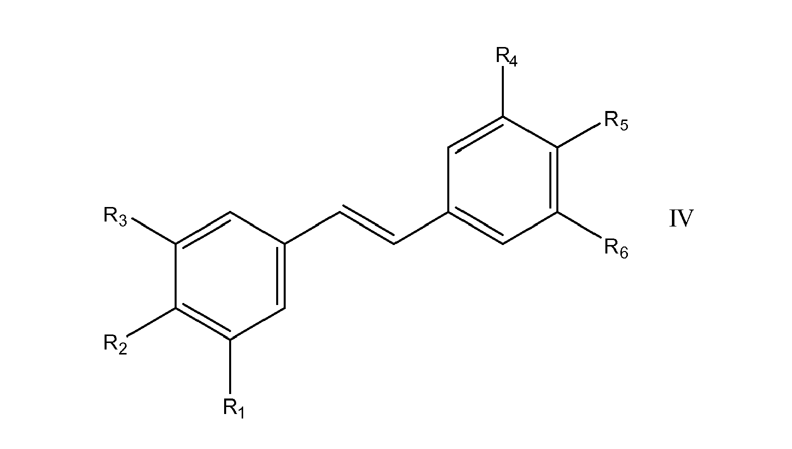
wherein
R₁, R₂, R₃, R₄, R₅ and R₆ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose; 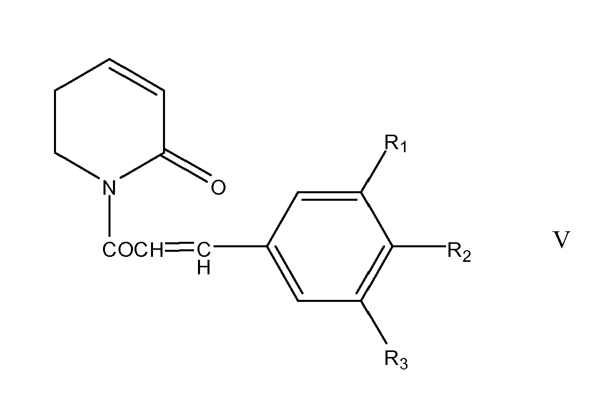
wherein
R₁, R₂, and R₃ are each independently selected from the group consisting of hydrogen, hydroxyl, -OCH₃, O-CH₂-CH₃, O-gallate and O-sugar, wherein the sugar is selected from the group consisting of glucose, maltose, gallactose and fructose; 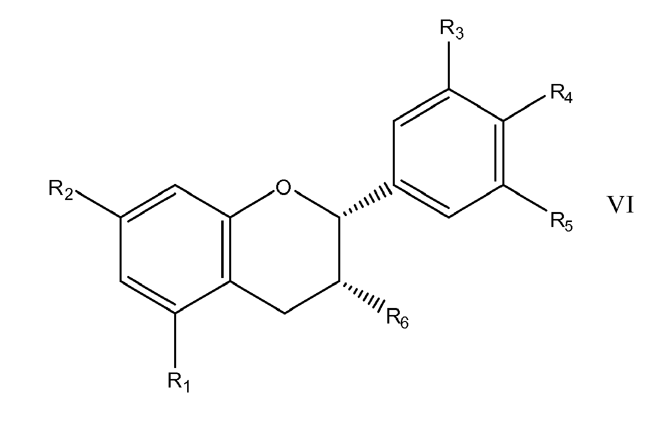
wherein
R₁, R₂ and R₆ are each independently selected from the group consisting of hydrogen, -OCH₃, O-CH₂-CH₃, O-gallate and O-sugar, wherein the sugar is selected from the group consisting of glucose, maltose, gallactose and fructose, and
wherein
R₃, R₄ and R₅ are each independently selected from the group consisting of hydrogen, O-CH₂-CH₃, O-gallate and O-sugar, wherein the sugar is selected from the group consisting of glucose, maltose, gallactose and fructose.

14. A compound for use according to claim 2, wherein the human suffers from depression and/or post-traumatic stress disorder, anterograde amnesia and/or retrograde amnesia, or navigation disorientation.

15. A compound for use according to claim 2, wherein the human is a soldier or combatant.
